# EUROPEAN PATENT APPLICATION

(11) **EP 2 377 510 A1**
(43) Date of publication of application: **19.10.2011**
(21) Application number: 09832974.1
(22) Date of filing: 30.11.2009
(51) Int. Cl.: A61K 8/34, A61K 8/97, C07C 37/82, A61K 36/63, A61K 36/28, A61K 36/185

(54) **METHOD FOR PURIFYING 3,4-DIHYDROXYPHENYLGLYCOL (DHPG) FROM PLANT PRODUCTS**

(30) Priority: 19.12.2008 ES 200803630
(71) Applicant: Consejo Superior de Investigaciones Cientificas (CSIC), 28006 Madrid (ES)
(72) Inventor: FERNÁNDEZ-BOLAÑOS GUZMÁN, Juan, E-41012 Sevilla (ES); GUILLÉN BEJARANO, Rafael, E-41012 Sevilla (ES); JIMENEZ ARAUJO, Ana, E-41012 Sevilla (ES); RODRÍGUEZ ARCOS, Rocío, E-41012 Sevilla (ES); RODRÍGUEZ GUTIÉRREZ, Guillermo, E-41012 Sevilla (ES); LAMA MUÑOZ, Antonio, E-41012 Sevilla (ES)
(74) Representative: Pons Ariño, Angel
(86) International application number: PCT/ES2009/070541
(87) International publication number: WO 2010/070168

(57) **Abstract**

The invention relates to a method for purifying 3,4-dihydroxyphenylglycol (DHPG) from any part of the plant, the products or the by-products obtained from the olive tree or any other plant product of the Oleaceae, Orobanchaceae, Plantaginaceae, Compositae, Lamiaceae, Acanthaceae and/or Scrophulariaceae families, including one or more steps in which the initial product is introduced into at least one ionic resin column, and possible subsequent steps in which the product eluted in the preceding column is introduced into a column containing ionic resin and/or non-ionic adsorption resin. The invention also relates to the DHPG extract which can be obtained by means of the aforementioned method and to the uses thereof.

## Description

This invention relates to a process for purifying 3,4-dihydroxyphenylglycol (DHPG) from any part of the plant, from products or by-products derived from olive trees or any other vegetable product of the families *Oleaceae, Orobanchaceae, Plantaginaceae, Compositae, Lamiaceae, Acanthaceae,* and/or *Scrophulariaceae.* Given the different applications of DHPG, this invention falls within the food, pharmaceutical or cosmetic sectors.

### PRIOR STATE OF THE ART

Epidemiological studies indicate that the Mediterranean diet is associated with a lower incidence of cardiovascular diseases, atherosclerosis and certain types of cancers (skin, breast, prostate, digestive tract). This diet is rich in fresh fruits and vegetables, with a relatively low proportion of animal fat and with olive oil as the main source of fat. These beneficial effects have been attributed not only to a low proportion of saturated/monounsaturated fatty acids in olive oil, but also to other additional molecules present at a lower concentration, in particular antioxidant phenolic compounds.

Interest in natural antioxidants is growing nowadays because there is increasing evidence that they are capable of counteracting the genotoxic and cytotoxic effects of free radicals and reactive oxygen species, which are produced in cases of oxidative stress and are involved in several pathological processes, such as kidney and liver diseases, and in inflammatory processes. This is why several of them are isolated from olives, the most significant of which is hydroxytyrosol (HT).

According to the "European Olive Oil Medical Information Library", HT is a phenolic compound with a special, well-known antioxidant effectiveness. Its antioxidant properties are attributed in particular to the presence of two hydroxyl groups in the ortho position, that is, an ortho-diphenol group, characteristic of biophenols.

In addition to its capacity for electron donation and free-radical neutralisation, its high antioxidant efficacy lies in its capacity for sequestering or chelating metal ions such as Fe or Cu, which are responsible for the formation of free radicals during the oxidation process. In recent years, it has been shown that this natural antioxidant also has important biological properties. *In vitro* and *in vivo* assays have demonstrated its capacity to act as a platelet anti-aggregating agent and as an inhibitor of cholesterol-rich low-density lipoprotein (LDL) oxidation, which would contribute to reducing atherosclerosis. HT is also capable of modulating cyclooxygenase, lipoxygenase and nitric oxide or NO-synthase, thereby contributing to palliate thrombogenic and inflammatory processes. Furthermore, it is capable of reducing the production of free radicals such as superoxide anion and, therefore, has an inhibitory effect on the initiation of mutagenic and carcinogenic processes, and induces apoptosis in HL-60 cells. Its capacity to act on Gram-negative and Gram-positive bacteria and on the HIV virus has also been demonstrated. In *in vivo* bioavailability studies in humans, conjugates of said compound and its metabolite, 3*-O*-methyl-hydroxytyrosol, have been detected in the plasma, the quantity absorbed being dependent on the ingested dose.

Consequently, currently there are numerous research studies underway which attempt to demonstrate this compound's performance in the prevention and treatment of a large number of diseases. It protects against neurodegenerative diseases, prevents ischaemic cerebral ictus and includes treatment of the skin. All these precedents suggest, moreover, the use of HT as a functional component in foods, as well as its use to prevent the deterioration thereof thanks to its demonstrated capacity to inhibit lipid oxidation.

On the other hand, 3,4-dihydroxyphenylglycol (DHPG) is a compound that is very similar to HT, but has an additional hydroxyl group. This substance has been described in olives, olive juice and olive oil.

Moreover, DHPG exhibits a high antioxidant activity, even higher than that of HT, its antioxidant efficacy in water being 2-3 times greater than that of ascorbic acid or HT, whereas, in a lipid medium, it is twice that of HT and similar to vitamin E (Rodríguez, G., Rodríguez, R., Fernández-Bolaños, J., Guillén, R., & Jiménez, A. (2007), European Food Research and Technology, 224, 733-741.) It is also worth noting that both HT and DHPG are a part of the same family, phenylpropanoid glucosides, also called acteosides or hydroxyacteosides, depending upon whether it is HT or DHPG which is a part of the molecule. The molecule is additionally made up of caffeic acid and a di- or trisaccharide, and is amply distributed as secondary metabolites in many plant species; it is widely used in traditional Oriental medicine (Chinese, Japanese and Korean) (Nishibe, S. (2002). Yakugaku Zasshi, 122, 363-379), due to its large variety of biological activities (anti-proliferative, anti-inflammatory, neurodegenerative, etc.), all of them attributable to its high antioxidant potency.

Due to its similarity with HT, and since it belongs to the phenylpropanoid glucosides family, it is desirable to develop a method for producing this substance, DHPG, whether pure or in an aqueous extract, with a high percentage of purity.

### DESCRIPTION OF THE INVENTION

This invention provides a process for obtaining a bioactive, natural concentrate starting from products and/or by-products derived from olive trees and/or any other vegetable product of the families *Oleaceae, Orobanchaceae, Plantaginaceae, Compositae, Lamiaceae, Acanthaceae,* and/or *Scrophulariaceae,* which contain DHPG, as well as for obtaining DHPG with a high degree of purity.

The compound obtained by means of the process of this invention is a phenolic extract which is very rich in DHPG.

DHPG is present, above all, in olives and virgin olive oil. It is also found in the human body, since it is a metabolite of the sympathomimetic hormone noradrenaline. It has a greater antioxidant capacity than its companion in olives, HT.

The process of the invention makes it possible to obtain DHPG with a high degree of purity using simple chromatographic techniques, wherein the absence of organic solvents substantially simplifies and reduces the costs, leading to a simple, very economical system which is fully compatible with food products.

The process for obtaining purified DHPG of this invention starts from products and by-products derived from olive trees and/or any other vegetable product of the families *Oleaceae, Orobanchaceae, Plantaginaceae, Compositae, Lamiaceae, Acanthaceae,* and/or *Scrophulariaceae.* The purification system is based on the use and combination of various chromatographic systems. Adsorption resins and ion-exchange resins, preferably strong or weak ion-exchange resins, are used. By combining series columns of these resins, a DHPG-rich solution is obtained.

The source of DHPG is introduced into said columns, to obtain, depending on the combination used, and following the elution thereof with water, a solution that contains DHPG with a purity of between 10%-100% by weight, together with other compounds such as tyrosol or HT, amongst others.

The process of the invention uses a chromatographic system in one, two, three or more phases, to obtain a natural product that to date has not been isolated. The number of phases to be used in this process of the invention will depend on the desired purity of the final product, DHPG.

Therefore, a first aspect of this invention relates to a process for obtaining purified DHPG (starting from products and by-products derived from olive trees and/or any other vegetable product of the family) from an initial product of selected vegetable species of the families *Oleaceae, Orobanchaceae,*
*Plantaginaceae, Compositae, Lamiaceae, Acanthaceae, Scrophulariaceae,* or any combination thereof, characterised in that it comprises (hereinafter process of the invention):
a. introducing the initial product into an ion-exchange resin column;
b. eluting the compound retained in the column of step (a).

In a preferred embodiment, the elution of step (b) is made to pass through a second column which contains a resin selected from an ion-exchange resin or a non-ionic adsorption resin and, subsequently, the retained compound is eluted.

In a more preferred embodiment of the process of the invention, the solution eluted from the second column is made to pass through a third column which contains a resin selected from an ion-exchange resin or a non-ionic adsorption resin and, subsequently, the retained compound is eluted.

In an even more preferred embodiment of the process of the invention, the solution eluted from the third column is made to pass through a fourth column which contains a resin selected from an ion-exchange resin or a non-ionic adsorption resin and, subsequently, the retained compound is eluted.

Preferably, any of the elutions of the product retained in any of the columns is performed with water.

Preferably, the first column uses an ion-exchange resin, which, in turn, may be a strong ion-exchange resin or a weak ion-exchange resin, which makes it possible to obtain a DHPG-rich solution following the elution thereof with water. This type of resin is preferably polystyrene-based, easily regenerable, with a great mechanical and functional durability, and low investment, regeneration and operating costs.

The second and third phases may use either an ion-exchange resin and a non-ionic adsorbent resin or a non-ionic adsorbent resin and an ion-exchange resin, respectively.

The adsorbent resins are preferably XAD polymeric; these are macroreticular, non-ionic, polystyrene-based resins, which adsorb and release the substances through hydrophobic and polar interactions.

In a preferred embodiment, the initial product used to obtain DHPG from vegetable species of the families *Oleaceae, Orobanchaceae, Plantaginaceae, Compositae, Lamiaceae, Acanthaceae,* and/or *Scrophulariaceae* comes from the whole plant, the aerial part, the leaves, the flowers, the seeds or the fruits.

Vegetable species of the families *Oleaceae, Orobanchaceae, Plantaginaceae, Compositae, Lamiaceae, Acanthaceae* and/or *Scrophulariaceae* are used in the process of the invention, since they include hydroxyacteosides.

In a preferred embodiment, the initial products used in the process of the invention are from vegetable species of the family *Oleaceae.* Examples of genera in this family include, amongst others, *Abeliophyllum, Chionanthus* L., *Comoranthus*, *Fontanesia* Labill., *Forestiera* Poir., *Forsythia* Vahl, *Menodora* Bonpl., *Myxopyrum, Nestegis* Raf., *Noronhia, Notelaea, Nyctanthes* or *Olea.* In a more preferred embodiment, the vegetable species are from the genus *Olea,* and, more preferably, from olive trees, their fruits, the oil extracted therefrom and the products or by-products derived from their industries, olive oil or table olives.

Preferably, the raw material comes from olive trees. The products or by-products derived from olive trees and the production of oil or olives may be selected from, without being limited thereto, two-phase olive pomaces (olive pulp), three-phase olive pomaces, olive juice, lyes, washing waters from olive seasoning, olive-tree leaves from olive processing, olive pits or any combination thereof.

Prior to being used in the process of the invention, these materials may be subjected to a pre-treatment, which may consist of a heat treatment or an extraction with organic solvents.

When pre-treatment of the initial product is performed by means of a thermal process, heating by direct or indirect contact using steam, gases or other heating liquids, or through electrical resistance, at a temperature of between 40°C and 200ºC, at a low or high pressure, with or without a steam explosion process.

Another preferred embodiment of the process of the invention additionally comprises the regeneration of the ion-exchange or adsorbent resin columns, to be re-utilised in the same process.

The regeneration of the ion-exchange columns consists of washing with a strong or weak, organic or inorganic, acid solution, such as, for example, without being limited thereto, sulphuric acid, phosphoric acid, hydrochloric acid, acetic acid, citric acid or nitric acid, and, following a large number of loading and unloading cycles, a prior washing with alkaline solution.

Preferably, the regeneration of the ion-exchange resin is performed by means of the following steps:
i. washing with a strong or weak, organic or inorganic basic solution, such as, for example, without being limited thereto, NaOH, KOH, NaOH/H₂O₂, pyridine, sodium, magnesium or aluminium carbonates; and
ii. washing with a strong or weak, organic or inorganic acid solution, such as, for example, without being limited thereto, sulphuric acid, phosphoric acid, hydrochloric acid, acetic acid, citric acid or nitric acid.

The regeneration of non-ionic adsorbent resin columns is performed by washing with an organic alcohol solution, such as, for example, ethanol or methanol, or other solvents such as acetonitrile, butanol, propanol, hexane, dichloromethane, trifluoromethane or chloroform. If necessary, it may additionally comprise washing with strongly basic solutions, such as, for example, without being limited thereto, NaOH, KOH, NaOH/H₂O₂, pyridine, sodium, magnesium or aluminium carbonates.

Another aspect of this invention relates to the extract that contains the DHPG obtained using the process of the invention, which has a purity of between 10% and 100% by dry weight. Preferably, a purity of between 50% and 100% by dry weight.

DHPG is a natural antioxidant that may be used in the preservation of food products and in the prevention of certain human diseases induced by free radicals during oxidative stress. In this regard, it may be used in the development of functional foods and to obtain various topical, anti-aging and anti-inflammatory preparations. Given its similarity to HT, with an additional hydroxyl group, and the fact that this compound exhibits a greater antioxidant capacity than HT itself, which has been shown to have interesting pharmacological effects, DHPG may play a favourable role in the prevention of cardiovascular, neurodegenerative and tumoural diseases.

Therefore, DHPG is an interesting compound, susceptible to being included within a healthy diet in order to protect against lipid oxidation induced by oxidative stress, which causes various pathologies that currently have a high impact on the population. Moreover, it may contribute to the promising, emerging role of antioxidants in general as therapeutic tools against neurodegenerative diseases such as Alzheimer's and Parkinson's diseases. The use of an inexpensive purified DHPG may also contribute to develop new routes for the preparation of biologically active products, thereby helping to produce new, cheaper drugs.

Therefore, another aspect of this invention relates to the use of the DHPG extract of the invention for the nutritional enrichment of foods; preferably, these foods are milk or the derivatives thereof, juices or fats.

Due to its antioxidant properties, the extract of the invention may be used in the treatment and/or prevention of diseases associated with oxidative stress. These diseases are known to those skilled in the art and may be selected from the list that comprises atherosclerosis, thrombogenic and inflammatory processes, mutagenic or carcinogenic processes, neurodegenerative diseases (for example, Alzheimer's disease), ischaemic cerebral ictus, amongst others.

Thus, another aspect of this invention relates to the use of the extract of the invention to prepare a pharmaceutical composition, preferably for the treatment and/or prevention of diseases associated with oxidative stress.

Another aspect of the invention relates to a pharmaceutical composition that comprises the extract of the invention, jointly with a pharmaceutically effective carrier.

"Pharmaceutically effective carrier" refers to the adjuvants and/or carriers known to those skilled in the art and habitually used in the preparation of therapeutic compositions.

Another aspect of this invention relates to a functional food that comprises the extract of the invention, for the prevention and/or treatment of diseases associated with oxidative stress.

In this invention, "functional food" is understood to mean those foods that are prepared not only for their nutritional characteristics, but also to perform a specific function, such as improving the health and reducing the risk of contracting diseases. To this end, biologically active components are added thereto; in this case, DHPG, which is an antioxidant, is added.

Other uses of the extract of the invention will arise from its antioxidant properties.

Throughout the description and the claims, the word "comprises" and the variants thereof are not intended to exclude other technical characteristics, additives, components or steps. For those skilled in the art, other objects, advantages and characteristics of the invention will arise partly from the description and partly from the practise of the invention. The following examples and drawings are provided for illustrative purposes, and are not intended to limit the scope of this invention.

### BRIEF DESCRIPTION OF THE FIGURES

**Fig. 1**. Diagram of four possible combinations of the process for purifying DHPG at the laboratory level.
**Fig. 2**. Diagram and system balance performed at the semi-industrial or pilot plant level.

### EXAMPLES

Below we will illustrate the invention by means of assays performed by the inventors, which show the effectiveness of the process of the invention.

The process for obtaining purified DHPG was developed starting from a sample of two-phase olive pomace subjected to a heat treatment. Two types of experiments were developed, one at the laboratory level, wherein four of the best combinations of the three types of resin were selected, and the other at the pilot plant or semi-industrial level. The latter was performed by selecting one of the best options on the basis of their simplicity, reproducibility and cost reduction.

### A) Laboratory-level experiment:

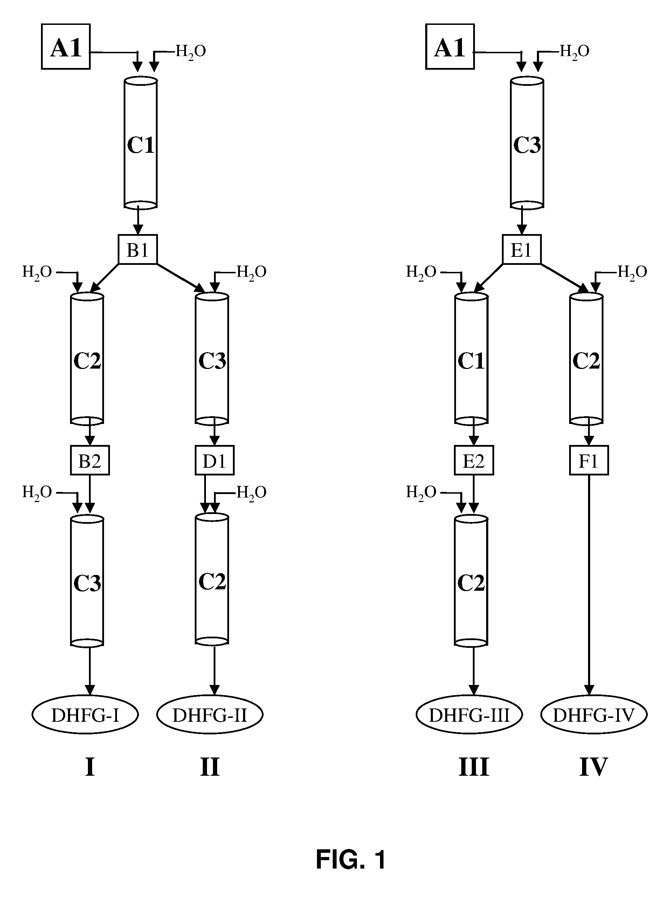

Approximately 7 kg of two-phase olive pomace were subjected to a heat treatment at 70ºC for two hours. Once completed, it was centrifuged to obtain a 4.75-I solution with a DHPG concentration of 0.2 g/I and a residual solid with 50% humidity, which means that there were 950 mg of said compound in the sample called A1. The experiment was repeated four times in order to perform the four diagrams in Figure 1:
A1: initial sample, source of DHPG (aqueous fraction).
C1: strong ion-exchange resin column wherein the compound is eluted, preferably with water.
C2: XAD-type adsorption column, wherein either only other compounds are retained, the DHPG being eluted with the loading liquor, or part or all of it must be eluted from the column, preferably with water.
C3: weak ion-exchange resin column, wherein the compound is eluted, preferably with water, and where the greatest leap in compound purity takes place.
DHPG I-IV: fractions of the purified compound with a range of purity of 10%-100%.

### Description of each diagram:

### Diagram I:

1st Step: Sample A1 was introduced into the first column (C1) with 1 l of strong ion-exchange resin. The DHPG was completely retained in the column. Washings were performed with water. The middle, purest fractions were joined, and about 360 mg, which were less purified, were discarded, together with about 136 mg that did not elute from the column. The resulting fraction (B1) was 7.85 I, with about 454 mg of DHPG with a purity of between 5% and 20% by dry weight.
2nd Step: Fraction B1 was made to pass through a column (C2) with 3 I of XAD-type resin, wherein the DHPG was completely retained. After elution with water, an 8.5-I fraction (B2) was obtained, containing 257 mg of the compound with a purity of 20%-30% by dry weight.
3rd Step: B2 was concentrated prior to being made to pass through the column (C3) with 0.5 I of weak ion-exchange resin, where a 150-mg fraction of DHPG-I, with a purity of 60%-80%, was obtained.

### Diagram II:

1st Step: Starting from the same sample, A1, it was made to pass through the same column (C1) as in the previous case, to obtain the same fraction B1.
2nd Step: In this case, fraction B1 was concentrated prior to being made to pass through a column (C3) with 0.9 I of weak ion-exchange resin. The concentrated fraction presented 412 mg of DHPG in 1.45 I. The DHPG was completely retained in the column. Following elution with water, a 5-I fraction (D1) was obtained which contained 300 mg of the compound with a purity of 15%-30% by dry weight.
3rd Step: D1 was made to pass through the column (C2) with 1.9 I of XAD-type adsorbent resin. Following elution with water, a fraction of 180 mg of DHPG-II, with a purity of 70%-90%, was obtained.

### Diagram III:

1st Step: Sample A1 was introduced into the first column (C3) with 2.6 of weak ion-exchange resin, wherein the DHPG was completely retained. Washings with water were performed. The middle, purest fractions were joined, and about 243 mg, which were less purified, were discarded, together with about 147 mg which did not elute from the column. The resulting fraction (E1) was 6.25 I, with about 560 mg of DHPG with a purity of between 5% and 20% by dry weight.
2nd Step: Fraction E1 was made to pass through column C1 with 1.9 I of strong ion-exchange resin, wherein the DHPG was completely retained. Following elution with water, an 11.36-I fraction (E2) was obtained, which contained 345 mg of the compound with a purity of 20%-40% by dry weight.
3rd Step: E2 was made to pass through the column (C2) with 2.3 I of XAD-type adsorbent resin. Following elution with water, a fraction of 273 mg of DHPG-III, with a purity of 70%-90%, was obtained.

### Diagram IV:

1st Step: The same experiment of the first step of diagram III was repeated, to obtain a resulting fraction (E1) of 6.25 I with 560 mg of DHPG, with a purity of between 5% and 20% by dry weight.
2nd Step: Fraction E1 was made to pass through a column (C2) with 3 I of XAD-type resin, wherein the DHPG was completely retained. Following elution with water, a 16-I fraction (F1) was obtained, which contained 537 mg of the compound with a purity of 50%-70% by dry weight.

Prior to each column, the solution containing the DHPG may be concentrated by reducing the quantity of resin to be used. The final solutions obtained may also be concentrated in order to increase the concentration thereof in said compound. Whenever a concentration operation is performed, a pH value of between 6 and 6.5 must be maintained, in order to prevent degradations. For the same reason, the product must be preserved at a pH always lower than 6.5, and, if possible, cooled or frozen.

### B) Semi-industrial level experiment:

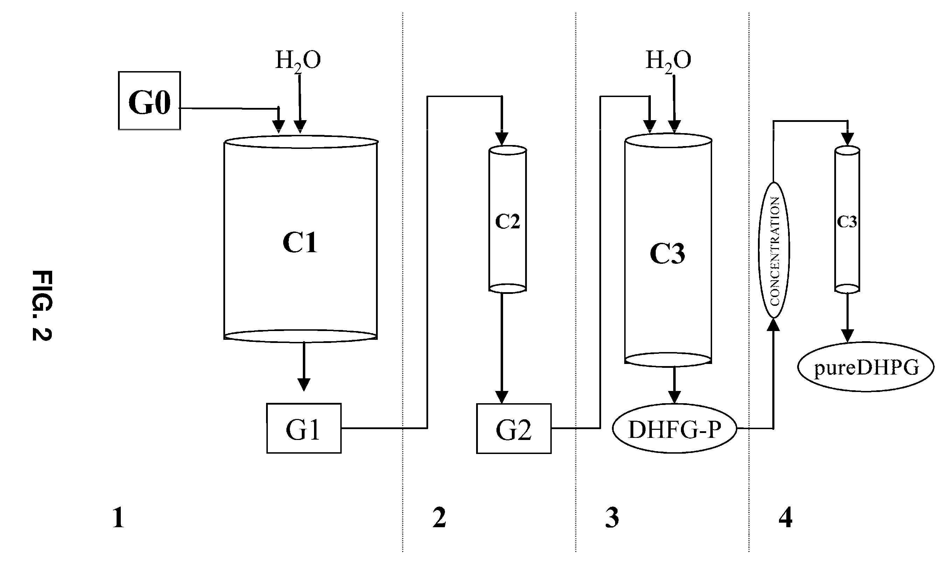

The experiment was performed starting from a liquid fraction obtained in the same manner as in the previous experiments, with a different two-phase olive pomace. On the basis of the results obtained in the laboratory, the optimal combination of resins was found to be that of Figure 2. This combination reduces the concentration steps, prevents the weak ion-exchange resin from coming into contact with large quantities of other phenolic compounds that hinder the regeneration thereof, and simplifies the size and use of the XAD-type resin. Using this diagram, the operating costs are reduced, which leads to a simple, economical system wherein water is the only eluent.

In Figure 2, C1, C2 and C3 are the same types of columns as in Figure 1.

About 250 kg of two-phase olive pomace were treated in several batches. Following centrifugation, a 150-I fraction (G0) was obtained, with a DHPG concentration of 0.45 g/I, which is the starting-point for this experiment (Figure 2):
1 st Step: Sample G0 was introduced into the first column (C1) with 90 I of strong ion-exchange resin. The DHPG was completely retained in the column. Washings with water were performed. The middle, purest fractions were joined, resulting in a 325-I fraction G1, with about 32 g of DHPG with a purity of between 5% and 20% by dry weight.
2nd Step: Fraction G1 was made to pass through a column (C2) with 20 I of XAD-type resin, wherein most of the DHPG was not retained. The same starting fraction was collected, plus 30 I of washing with water, to obtain a 355-I fraction G2, with 25 g of the compound with a purity of between 20%-40% by dry weight.
3rd Step: G2 was made to pass through the column (C3) with 30 I of weak ion-exchange resin, wherein the whole compound was retained, and, following elution with water, a 235-I fraction of DHPG-P was obtained, which contained 15 g of DHPG with a purity of 80%-95%.
4th Step: In order to increase the purity of this last fraction, the DHPG-P was concentrated to 10 I with 13 g of the compound. The concentrate was made to pass through a column (C3) with 2 I of weak anionic resin. The compound was completely retained and finally, following elution with water, a 10-g fraction of pure DHPG was obtained, with a purity ranging between 95%-100% by dry weight.

## Claims

1. Process for obtaining purified DHPG from an initial product from vegetable species selected of the families *Oleaceae, Orobanchaceae, Plantaginaceae, Compositae, Lamiaceae, Acanthaceae, Scrophulariaceae* or the combinations thereof, **characterised in that** it comprises:
a. introducing the initial product into an ion-exchange resin column;
b. eluting the compound retained in the column of step (a).

2. Process for obtaining DHPG according to claim 1, where the solution eluted in step (b) is made to pass through a second column, which contains a resin selected from an ion-exchange resin or a non-ionic adsorption resin and, subsequently, the retained compound is eluted.

3. Process for obtaining DHPG according to claim 2, where the solution eluted from the second column is made to pass through a third column, which contains a resin selected from an ion-exchange resin or a non-ionic adsorption resin and, subsequently, the retained compound is eluted.

4. Process for obtaining DHPG according to claim 3, where the solution eluted from the third column is made to pass through a fourth column, which contains a resin selected from an ion-exchange resin or a non-ionic adsorption resin and, subsequently, the retained compound is eluted.

5. Process for obtaining DHPG as claimed in any of claims 1 to 4, where the elution of the product retained in the column is performed with water.

6. Process as claimed in any of claims 1 to 5, where the ion-exchange resin is a strong ion-exchange resin or a weak ion-exchange resin.

7. Process for obtaining DHPG as claimed in any of claims 1 to 5, where the non-ionic adsorption resin is of the XAD type.

8. Process for obtaining DHPG as claimed in any of claims 1 to 7, where the initial product from the vegetable species of the families *Oleaceae, Orobanchaceae, Plantaginaceae, Compositae, Lamiaceae, Acanthaceae* or *Scrophulariaceae* is from the entire plant, the aerial part, the leaves, the flowers, the seeds or the fruits.

9. Process for obtaining DHPG as claimed in any of claims 1 to 8, where the initial product comes from olive trees or from the products and/or by-products obtained from the production of olive oil.

10. Process for obtaining DHPG according to claim 9, where the initial product is two-phase olive pomace (olive pulp).

11. Process for obtaining DHPG according to claim 9, where the initial product is three-phase olive pomace, the olive juices thereof, or a combination of both.

12. Process for obtaining DHPG according to claim 9, where the initial product are olive pits.

13. Process for obtaining DHPG according to claim 9, where the initial product are olive-tree leaves, branches, wood or any combination thereof.

14. Process for obtaining DHPG according to claim 9, where the initial product are the solutions of the elaboration process of olives for food.

15. Process for obtaining DHPG according to any of claims 1 to 14, where the initial product is subjected to a pre-treatment prior to being introduced into the first column.

16. Process for obtaining DHPG according to claim 15, where the pre-treatment is a heat process or an aqueous or organic extraction.

17. Process for obtaining DHPG according to claim 16, where the thermal process is a heating process by direct or indirect contact using steam, gases or other heating liquids, or through an electrical resistance, at a temperature of between 40ºC and 200ºC.

18. Process for obtaining DHPG according to any of claims 1 to 17, which additionally comprises the regeneration of the ion-exchange or absorbent resin columns, to be re-utilised in the same process.

19. Process for obtaining DHPG according to claim 18, where regeneration of the ion-exchange resin is performed by washing with an acid solution.

20. Process for obtaining DHPG according to claim 19, where the acid solution is a sulphuric acid, phosphoric acid, hydrochloric acid, acetic acid, citric acid or nitric acid solution.

21. Process for obtaining DHPG according to any of claims 19 or 20, where, prior to the washing in an acid solution, a washing is performed with a basic solution.

22. Process for obtaining DHPG according to claim 18, where regeneration of the adsorbent resin is performed by washing with aqueous solutions that contain organic solvents.

23. Process for obtaining DHPG according to claim 22, where the organic solvents are selected from the list that comprises ethanol, methanol, acetonitrile, butanol, propanol, hexane, dichloromethane, trifluoromethane or chloroform.

24. Process for obtaining DHPG according to any of claims 22 or 23, where regeneration of the adsorbent resin additionally comprises washing with a basic solution.

25. Process for obtaining DHPG according to any of claims 21 or 24, where the basic solution is a NaOH, KOH, NaOH/H₂O₂, pyridine or sodium, magnesium or aluminium carbonate solution.

26. DHPG extract obtainable by means of the Process according to any of claims 1 to 25, with a purity of between 10% and 100% richness by total dry weight.

27. Extract according to claim 26, where DHPG has a purity of between 50% and 90% richness by total dry weight.

28. Use of the extract according to any of claims 26 or 27, for the nutritional enrichment of foods.

29. Use according to claim 28, where the foods are selected from milk or the derivatives thereof, juices or fats.

30. Use of the extract according to any of claims 26 or 27, for the preparation of a pharmaceutical composition.

31. Use of the extract according to claim 30, for the treatment and/or prevention of diseases associated with oxidative stress.

32. Pharmaceutical composition that comprises an extract according to any of claims 26 or 27, jointly with a pharmaceutically effective carrier.

33. Functional food that comprises an extract according to any of claims 26 or 27, for the prevention and/or treatment of diseases associated with oxidative stress.
